# EUROPEAN PATENT APPLICATION

(11) **EP 3 811 857 A1**
(43) Date of publication of application: **28.04.2021**
(21) Application number: 20151678.8
(22) Date of filing: 14.01.2020
(51) Int. Cl.: A61B 5/0205, A61B 5/021, A61B 5/024, A61B 5/00

(54) **SYSTEM AND METHOD FOR PROCESSING PHYSIOLOGICAL SIGNALS TO DETERMINE HEALTH-RELATED INFORMATION**

(30) Priority: 24.10.2019 US 201962925376 P
(71) Applicant: Koninklijke Philips N.V., 5656 AG Eindhoven (NL)
(72) Inventor: ROMANO, Robert, 5656 AE Eindhoven (NL)
(74) Representative: Steenbeek, Leonardus Johannes

(57) **Abstract**

A system and method for managing the care of a patient includes receiving physiological signals of the patient, extracting first information from the physiological signals, extracting second information from the physiological signals, correlating the first information to the second information, and determining a condition of the patient based on the correlation between the first information and the second information. The first information may include a systemic parameter such a pulse pressure. The second information may include respiration information. The condition may be blood pressure or another vital sign of the patient. Correlating pulse pressure to one or more phases of respiration may reduce distortion in the blood pressure reading as a result of pulsus paradoxus.

## Description

### TECHNICAL FIELD

This disclosure relates generally to processing information, and more specifically, but not exclusively, to processing physiological signals to determine health-related information.

### BACKGROUND

Vital signs provide an empirical basis for determining patient health and also aids in making diagnosis and treatment decisions. The vital signs that are most often measured in any clinical setting include pulse, blood pressure, temperature, and respiration rate. These signs provide useful insight into a variety of adverse health conditions including arrhythmias, hypertension, and infection, just to name a few. For example, when it comes to blood pressure, studies have shown that more than 20% of adults have hypertension. This is a major risk factor for heart disease and stroke.

However, existing devices and methods for measuring vital signals have often proven to be inaccurate. This may be caused by various factors. For example, blood pressure may naturally fluctuate in a person due to respiration. These fluctuations can often frustrate the ability to obtain a consistent, true blood pressure reading. This, in turn, can lead to misdiagnoses or improper medication dosages.

### SUMMARY

It is an object of the invention to provide an improved system and method for processing physiological signals to determine health-related information. The invention is defined by the independent claims. The dependent claims define advantageous embodiments.

A brief summary of various example embodiments is presented below. Some simplifications and omissions may be made in the following summary, which is intended to highlight and introduce some aspects of the various example embodiments, but not to limit the scope of the invention. Detailed descriptions of example embodiments adequate to allow those of ordinary skill in the art to make and use the inventive concepts will follow in later sections.

Embodiments of the invention provide a system and method for managing the care of a patient includes receiving physiological signals of the patient, extracting first information from the physiological signals, extracting second information from the physiological signals, correlating the first information to the second information, and determining a condition of the patient based on the correlation between the first information and the second information. The first information may include a systemic parameter such a pulse pressure. The second information may include respiration information. The condition may be blood pressure or another vital sign of the patient. Correlating pulse pressure to one or more phases of respiration may reduce distortion in the blood pressure reading as a result of pulsus paradoxus.

### BRIEF DESCRIPTION OF THE DRAWINGS

The accompanying figures, where like reference numerals refer to identical or functionally similar elements throughout the separate views, together with the detailed description below, are incorporated in and form part of the specification, and serve to further illustrate example embodiments of concepts found in the claims and explain various principles and advantages of those embodiments.

These and other more detailed and specific features are more fully disclosed in the following specification, reference being had to the accompanying drawings, in which:
FIG. 1 illustrates an example of pulsus paradoxus in the pressure pulse signals of a patient;
FIG. 2 illustrates an embodiment of system for measuring one or more vital signs of a patient;
FIG. 3 illustrates an embodiment of system for measuring one or more vital signs of a patient;
FIG. 4A illustrates an example of a flow diagram of the method and FIG. 4B illustrates an example of a waveform analysis relating to blood pressure;
FIG. 5 illustrates an example of pulse pressure correlated to respiration information;
FIG. 6 illustrate an example of waveforms correlated to respiration phases from which vital signs may be computed;
FIG. 7 illustrates an embodiment of a method for measuring one or more vital signs;
FIG. 8 illustrates an example of an R wave relating to pulse transit time;
FIG. 9 illustrates an example of a pulse pressure labeled with blood pressure features; and
FIG. 10 illustrates an embodiment of a method for measuring one or more vital signs.

### DETAILED DESCRIPTION OF EMBODIMENTS

It should be understood that the figures are merely schematic and are not drawn to scale. It should also be understood that the same reference numerals are used throughout the figures to indicate the same or similar parts.

The descriptions and drawings illustrate the principles of various example embodiments. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or (i.e., and/or), unless otherwise indicated (e.g., "or else" or "or in the alternative"). Also, the various example embodiments described herein are not necessarily mutually exclusive, as some example embodiments can be combined with one or more other example embodiments to form new example embodiments. Descriptors such as "first," "second," "third," etc., are not meant to limit the order of elements discussed, are used to distinguish one element from the next, and are generally interchangeable. Values such as maximum or minimum may be predetermined and set to different values based on the application.

Example embodiments describe a system and method for measuring vital signals in a way that is more accurate than other methods and which allows a greater amount of information to be extracted that is relevant to patient health. The system and method may be applied to measure and extract information from a variety of vital signs. The example of blood pressure is discussed extensively below, but the embodiments described herein may be applied to other vital signs including, but not limited to, heart rate and heart rate variability.

### Inaccurate Blood Pressure Measurement

Blood pressure and its indices (systolic pressure, diastolic pressure and mean pressure) provide valuable medical information about the cardiovascular system. Several methods are used to measure representative systemic arterial blood pressure. These methods include direct and indirect methods. An example of a direct invasive method involves measuring blood pressure using an indwelling, usually radial arterial catheter. Indirect methods may be performed using non-invasive techniques. One example of a non-invasive technique involves using a sphygmomanometer that relies on auscultation (e.g., using a stethoscope and a cuff-based device which interrupts regular blood flow). Another example of a non-invasive technique involves using an automated system that includes an occlusion cuff and microphone.

Other methods involve analyzing signals generated by pulsatile waveform sensors to derive physiological parameters. One type of pulsatile waveform sensor is a photo-optical sensor that illuminates the skin and calculates light reflected or transmitted signals as absorption changes. The output of such a sensor may be used to generate a PhotoPlethysmoGram (PPG). Using the PPG, pressure pulses caused by vascular volume changes may be detected in order to provide an indication of systemic arterial blood pressure. This method may prove to be more accurate than the other types of methods described above, at least in certain circumstances.

All of the aforementioned blood pressure measurement methods suffer from systematic methodological error. This type of error is caused by pulsus paradoxus, a physiological phenomenon that occurs during a specific phase of respiration. In health individuals, systolic blood pressure falls by a certain amount (e.g., less than 10 mmHg) during quiet inspiration. However, in some people, an abnormally large decrease in stroke volume, systolic blood pressure, and pulse wave amplitude occurs during inspiration. When the drop of systolic blood pressure falls by more than 10 mmHg during the inspiration phase of respiration, it is referred to as pulsus paradoxus. Pulsus paradoxus is the principal cause of the fluctuation in pressure pulse that occurs in a human.

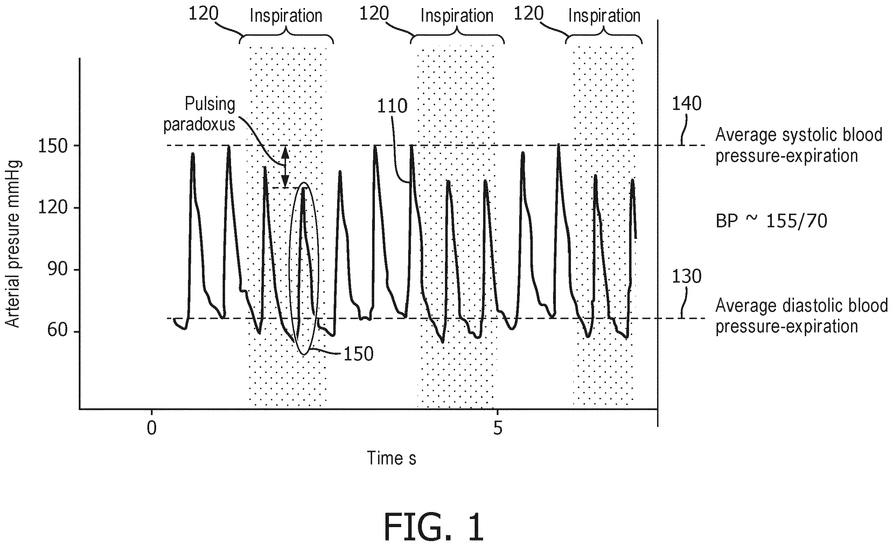

FIG. 1 illustrates an example of an example of pulsus paradoxus that may be found in the pulse pressure signals of a patient over one or more phases of respiration. In FIG. 1, a pressure pulse waveform 110 is included in a graph which plots arterial pressure (mmHg) along the vertical axis against time (seconds) along the horizontal axis, and areas 120 indicate phases of inspiration (inhalation). The pressure pulse waveform has peaks and troughs that mostly lie within a predetermined arterial pressure range, which in this example is between 65 mmHg and 152 mmHg.

More specifically, the lower limit 130 of the range corresponds to average diastolic blood pressure during expiration (exhalation) and the upper limit 140 of the range corresponds to average systolic blood pressure during expiration. Thus, the positive slopes in the waveform represent the systolic upstroke, the positive peaks the systolic peak pressure, and declining slope the diastolic decline, and the negative trough the diastolic pressure. (The waveform also includes dicrotic notches at points along the declining slopes of respective diastolic declines). The patient having the waveform of FIG. 1 has a blood pressure of about 155/70.

The graph also shows an aberration in the waveform caused by pulsus paradoxus. This aberration corresponds to the portion in circle 150 and represents a drop in systolic pressure of more than 10 mmHg occurs relative to the adjacent peaks. This portion of the waveform therefore represents a fluctuation that is indicative of pulsus paradoxus. More specifically, in the graph, pulsus paradoxus may be readily observed in conditions where intrathoracic pressure swings are exaggerated or the right ventricle is distended, which may occur, for example, in cases of severe acute asthma or exacerbations of chronic obstructive pulmonary disease. In patients with airway obstruction or cardiovascular disease, this difference may be greater than 20 mmHg or even 30 to 40 mmHg in severe conditions.

Because of the natural modulation that occurs in a blood pressure reading, the ability to obtain a consistent reading can prove to be challenging using other methods which have been proposed. Moreover, other methods which have been proposed do not take the effects of pulsus paradoxus into account when attempting to measure blood pressure. This leads to further inconsistencies and inaccuracies that ultimately may inure to the detriment of the patient. As a consequence, patients may be misdiagnosed or prescribed wrong or improper dosages of medicine.

Attempts have been made to improve the accuracy of the blood pressure readings taken by these proposed methods. One approach involves taking the average of data in the waveform across many respiratory cycles. However, this only serves to create a consistent bias in the error and does nothing to account for fluctuations caused by pulsus paradoxus.

In accordance with one or more embodiments, a system and method for determining blood pressure readings in a way that compensates for pulsus paradoxus is provided. Moreover, in these or other embodiments, the error in blood pressure readings caused by pulsus paradoxus is filtered out or otherwise differentiated from the modulation error caused by so-called Mayer waves, which occur during states of sympathetic activation and at a frequency lower than respiration rate. As a result, the embodiments described herein provide blood pressure measurements with improved accuracy compared with other methods which have been proposed.

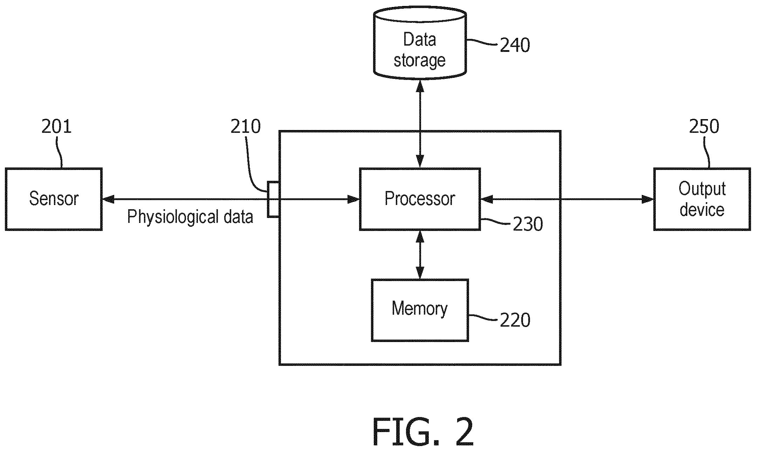

FIG. 2 illustrates an embodiment of a system for measuring one or more vital signs of a patient. The system includes an input 210, a memory 220, a processor 230, a data storage area 240, and an output device 250. The input 210 may be an interface that is coupled to one or more sensors 201 through a wired or wireless connection. The type of sensor matches the physiological signals to be captured for the patient. For example, when the physiological signals are pulse pressure signals, the sensor 201 may be any of the sensor types previously described. The output of the sensor may be received as a waveform or may be in the form of raw signals that are to be processed into a waveform (e.g., with optional filtering or other preprocessing operations). The processing may be performed, for example, by the sensor itself or by processor 230.

The memory 220 may be a non-transitory computer-readable medium that stores instructions for implementing the embodiments described herein for measuring the vital sign(s) of a patient. The instructions may be in the form of firmware, operating system software, or an application that may be found, for example, on a computing device. The memory may be, for example, a read-only memory (ROM), random-access memory (RAM), magnetic disk storage media, optical storage media, flash memory, or another type of storage medium. As such, the memory 220 and processor 230 may be included in a notebook computer, mobile computing device, cloud-computing system, smartphone, network processor, server, or another type of processing logic. In some embodiments, the memory 220 may be at a remote location and connected to the processor 230 through a network.

The processor 230 executes the instructions stored in the memory 220 to process and measure one or more vital signs of the patient based on the physiological signals. The processing and measurement operations may be performed in accordance with the method embodiments described herein. In one embodiment, the processor may implement a machine-learning algorithm or other model trained to generate an accurate indication of the vital sign(s) of interest based on the physiological signals from the sensor(s). Embodiments of the algorithms/models implemented by the processor 230 are discussed in greater detail below.

The data storage 240 may be a database or other storage area included in the same device or system as the processor 230 or coupled to the processor 230 through a wired or wireless link. In one embodiment, the data storage may be a cloud storage device, server, computer system of a hospital or other medical facility, or another type of database or storage area. The data storage 240 may store the physiological signals, waveforms, vital sign measurements, and the results of any additional processing performed, for example, to diagnose, detect, and/or predict the condition of the patient based on the vital sign measures. In implementation, these results may be stored in electronic medical records for the patient, that may be made available, upon request, by medical personnel on an as needed basis. The information in the data storage may be encrypted and/or may be stored in a blockchain to protect the privacy interests of the patient.

The output device 250 provides an indication of the vital sign measurements and other processing results generated by the processor 230. The output device may be, for example, a display, meter, or other form of analog or digital output device. In one embodiment, the memory 220, processor 230, and output device 250 may be incorporated within a single device. In this case, all or a portion the embodiments may be implemented as an application on a smartphone or a specially designed mobile or transportable device. In other embodiment, the output device 250 may be remotely located from the sensor and/or processor. In this case, the processor may communicate with the output device through a network connection, which may be a local wireless connection or a wide area network connection coupled to the processor through a wired or wireless link.

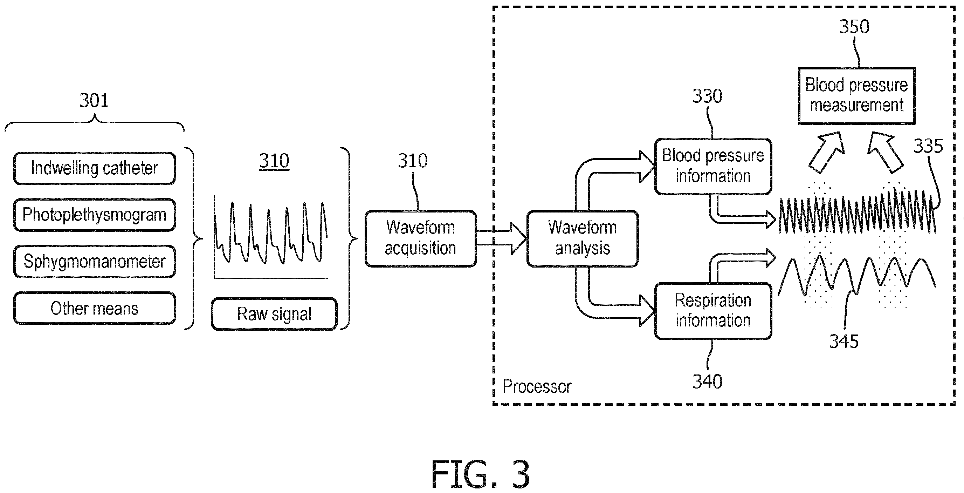

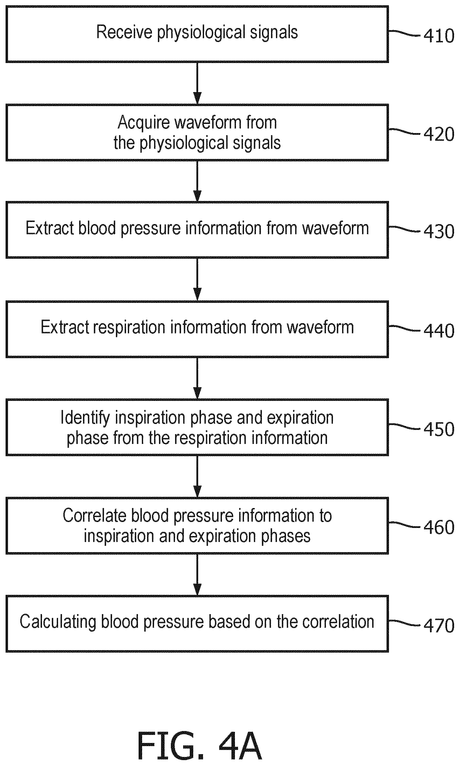

FIG. 3 illustrates an embodiment of a method for measuring one or more vital signs of a patent based on physiological signals. This method embodiment may be implemented by the system of FIG. 2 or a different system. FIG. 4A illustrates a textual description of operations of the method.

Referring to FIGS. 3 and 4A, at 410, physiological signals 310 are received from one or more sensors. The signals may be received through input 210 of the system and are indicative of one or more vital signs to be measured for the patient. In one embodiment dedicated to measuring blood pressure, the physiological signals 310 may include pulse pressure signals obtained from one or more blood pressure sensors 301. Examples of the blood pressure sensors include an indwelling catheter, a PPG sensor, a sphygmomanometer, or another type of sensor for capturing pulse pressure signals. When the embodiments are applied to determine a vital sign different from blood pressure, the physiological signals may be received from a corresponding type of sensor.

For illustrative purposes, it will be assumed that the physiological signals 310 are pulse pressure signals that are to be processed in order to determine an accurate blood pressure reading. The pulse pressure signals may be captured, for example, by direct pressure measurement of the sensor or based on a blood pressure pulse obtained from changes in blood volume detected by the sensor.

At 420, in one embodiment an input waveform 320 is acquired based on the raw physiological signals 310 received from the sensor(s) 301. While the raw signals 310 and waveform acquisition is shown in FIG. 3 as being performed external to the sensors, in one embodiment the waveform acquisition may be performed by processing logic in the sensor(s) from the raw signals. In another embodiment, intervening processing logic or processor 230 may receive and pre-process the raw signals to derive a waveform corresponding to the physiological sensor signals. The waveform may represent a discrete or continuous representation of the pulse pressure signals.

At 430 and 440, the processor 230 performs an analysis of the input waveform 320 in accordance with the algorithm embodied within the instructions stored in memory 220. This analysis involves extracting predetermined types of information from the input waveform. For example, when the embodiments are used to measure blood pressure, operation 430 includes extracting blood pressure information 330 from the input waveform and, at 440, extracting respiration information 340 from the input waveform by the processor 230.

The blood pressure information 330 may include features such as peaks corresponding to systolic blood pressure, peaks corresponding to diastolic blood pressure, the time interval(s) between adjacent peaks in the waveform, and the location of dicrotic notches in the waveform, as well as other features. The processor may analyze the input waveform to extract these features using various ways. For example, blood pressure may be determined based on the peak and the trough of a signal from a corresponding sensor, where the peak is representative of the systolic blood pressure and the trough is representative of the diastolic blood pressure. Blood pressure may also be determined by analysis of the area under the curve of a pressure pulse signal, with portions of the width corresponding to the systolic and diastolic pressures. In yet another method, the blood pressure may be determined based on calculations related to the slope of the ascending and/or descending portions of the pressure pulse curve. According to another method, the blood pressure may be determined based on dissection of various portions of the blood pressure pulse that take the dichotic notch into consideration. According to another method, blood pressure may be determined using a pattern recognition algorithm or a machine-learning algorithm. The result of the waveform analysis may be a waveform 335 that corresponds to or is based on the input waveform. The identification of these features allow for an accurate blood pressure measurement to be derived when taken relative to the respiration information.

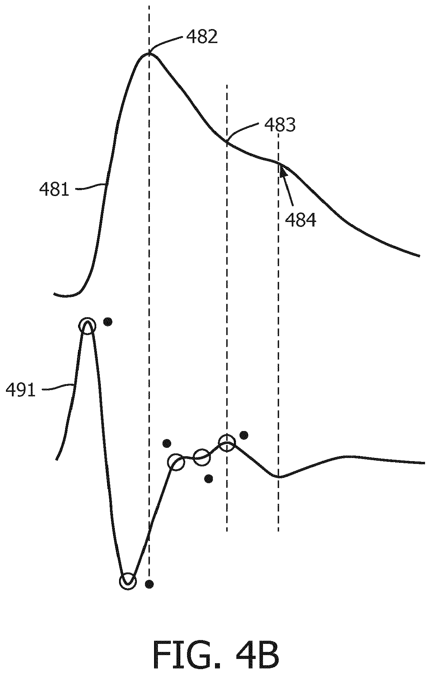

FIG. 4B illustrates an example of the waveform analysis performed using one or more of the aforementioned example methods. This analysis may involve determining blood pressure based on the first derivative or the second derivative of the curves in the waveform, or additional methods of calculus may be performed upon the signal, as well as ratios of segments. The example methods may be understood, for example, with respect to at least one of the two waveforms 481 and 491. Waveform 481 includes features indicating systolic peak 482, dicrotic notch 483, and diastolic peak 484. Waveform 491 is representative of a second derivative wave of the pressure pulse signal of a PPG with corresponding points based on waveform 481.

The respiration information 340 includes the different phases of respiration, namely the inspiration (inhalation) phase and the expiration (exhalation) phase. The different phases of respiration information may be extracted from the input waveform in various ways. Respiration normally occurs at a much lower frequency than heart rate and imposes amplitude modulation (pulsus paradoxus) upon the pressure pulse signal. Therefore, one way the different phases of respiration information may be extracted involves separating the low frequency components of the pulse pressure waveform from the high frequency components. The low frequency components may correspond to a respiration waveform and the high frequency components, for example, to blood pressure. Various signal analysis techniques may be used to extract the respiration information from the pulse pressure signal, including but not limited to pattern recognition methods and machine-learning algorithms. The result may be a waveform 345 with peaks and valleys that may have a near periodic repetitive pattern or a different pattern, for example, depending the pulmonary condition of the patient.

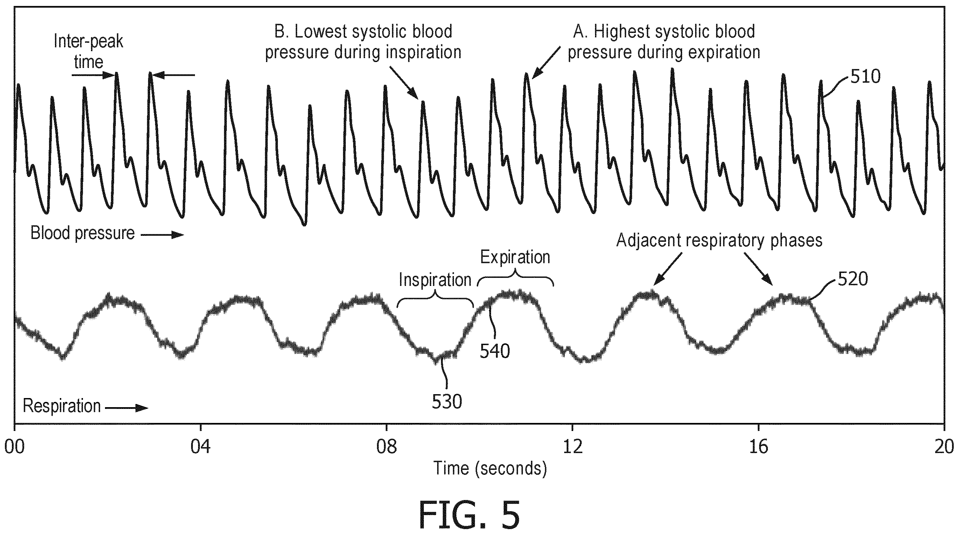

FIG. 5 illustrates examples of the blood pressure information and respiration information that may be extracted or identified by the processor 230 from the input waveform. The blood pressure information may be represented by a waveform 510 (e.g., corresponding to waveform 335) and the respiration information may be represented by waveform 520 (e.g., corresponding to waveform 345).

At 450, the respiration information may be analyzed by the processor 230 to determine different phases of the respiration waveform, namely the inspiration (inhalation) phase and the expiration (exhalation) phase. A portion of the respiration waveform that coincides with the inspiration phase is labeled 530. A portion of the respiration waveform that coincides with the expiration phase is labeled 540. In general, the positive peaks correspond to the expiration phase and the negative peaks correspond to the inspiration phase, with these two phases being adjacent to one another.

In one embodiment, the processor 230 may detect the inspiration and expiration phases of respiration based on the pulsus paradoxus modulations that occur in the blood pressure information (e.g., waveform). Pulsus paradoxus modulation may correspond to the respiration modulation, e.g., the respiration modulation is caused by pulsus paradoxus. Thus, in accordance with one embodiment, the pulsus paradoxus phenomena may be used as a basis for determining respiration. An example of a pulsus paradoxus modulation in an input waveform is labeled by reference numeral 150.

In one embodiment, the respiration phases may be determined by applying methods of calculus to determine peaks and troughs in varying signals (of the pulse pressure waveform). Since there is a likelihood of random noise within the signal, the signal may be pre-processed using filters (either hardware or software) or more advanced calculus methods may be used such as taking the second derivative or least squares curve fitting. Likewise, pattern recognition and machine learning can be used in order to determine respiration phases. Detecting the different phases of respiration may be performed, for example, based on a neural network, a frequency domain transformation, an amplitude modulation decomposition technique, or using another mathematical algorithm or model.

At 460, the temporal and spatial relationship between the blood pressure information and the respiration information is determined over time. This may involve the processor 230 correlating different portions of the blood pressure waveform to (e.g., that occur simultaneously or coincidentally with) the inspiration and expiration phases of the respiration waveform. The portions of the blood pressure waveform that coincide with the inspiration phases of respiration may be determined, for example, from a single or average of several observations of the data obtained that correlates to the blood pressure in one respiratory phase or a series of averages or of median values of the pulsations across several (typically adjacent) phases. The portions of the blood pressure waveform that coincide with the expiration phases of respiration.

At 470, the following operations are performed to calculate an accurately blood pressure measurement 350 based on the temporal and spatial correlation between the blood pressure information and the respiration information. For example, the blood pressure measurement may be calculated based on the blood pressure information occurring during the inspiration phase of the respiratory cycle only or the expiration phase of the respiratory cycle, or both.

In one embodiment, the blood pressure measurement may be calculated based on a differential of the blood pressure information taken between the inspiration and expiration phases of the respiratory cycle.

### Additional Embodiments

The system and method previously described may be applied to calculate other vital sign or physiological measurements in a way more accurately than other techniques have been proposed. Like the blood pressure-related embodiments, these additional embodiment achieve, at least in part, the improved accuracy by correlating sensor signals with one or more of the different phases of respiration.

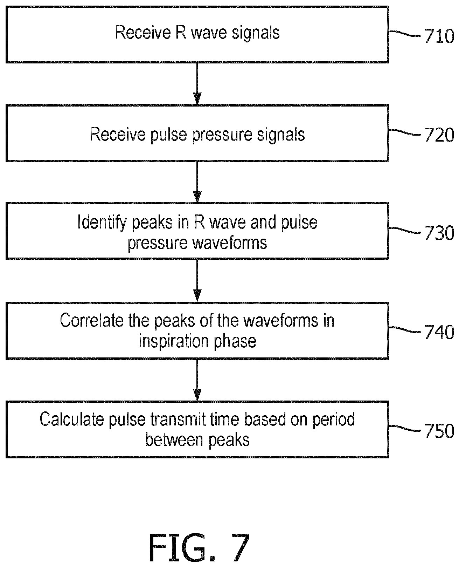

FIG. 7 illustrates an embodiment of a method for determining other types of vital signs or physiological measurements of a patient. The method may be performed, in whole or part, for example, by the system of FIG. 2 or may be performed by another method. In one embodiment, the system and method may be applied to determine the pulse transit time of a patient, e.g., the time it takes a pulse pressure wave to travel between two arterial sites. The speed at which this arterial pressure wave travels is directly proportional to blood pressure.

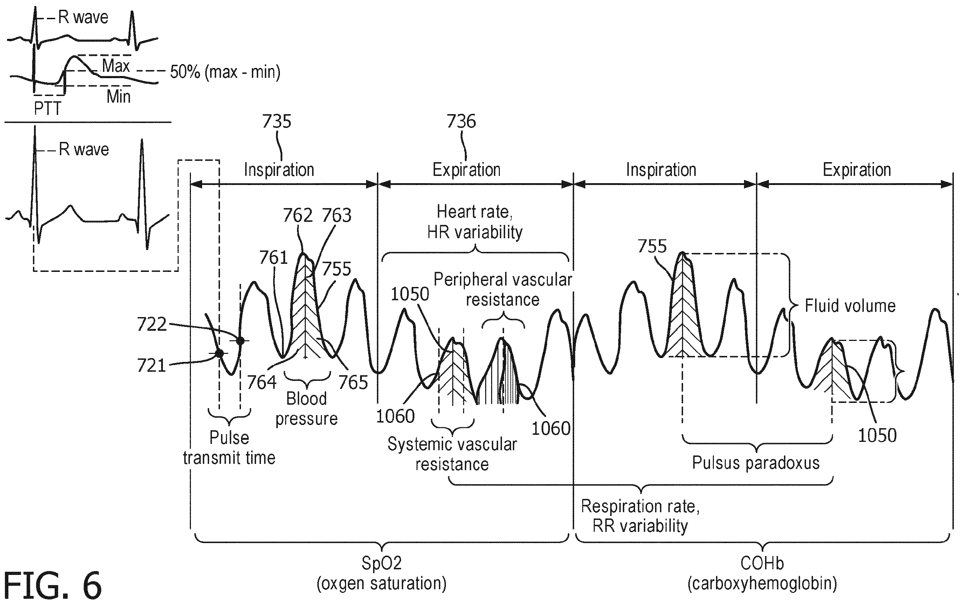

Referring to FIGS. 6, 7, and 8, the method may include, at 710, receiving physiological signals from a first sensor that monitors the R wave 711 of a patient. The first sensor may detect the R wave as the first upward deflection after the P wave as part of the QRS complex. Such a sensor may correspond, for example, to two electrodes in a twelve-lead arrangement used to capture an electrocardiogram of the patient.

At 720, the method may include receiving pulse pressure signals corresponding to waveform 712 obtained, for example, by a second sensor which may be a fingertip sensor.

At 730, once both waveforms have been obtained, the peaks in waveforms 711 and 712 (which usually overlap) are identified.

At 740, the peaks are correlated to a portion of the input waveform of the pulse pressure signal (e.g., waveform 320 in FIG. 3) that corresponds to a predetermined phase of the respiration information generated from the blood pressure information (e.g., as previously described). The respiration information may be obtained, for example, during SpO₂ saturation and/or COHb (carboxyhemoglobin) phase. In one embodiment, the predetermined phase may be the inspiration phase 735, as shown in FIG. 6. In another embodiment, the predetermined phase may be expiration.

At 750, based on this correlation, the pulse transmit time may be computed based on the period of time between the peaks projected onto the pulse pressure waveform of FIG. 6. This time period may be, for example, e.g., the time period between point 721 and point 722 on the portion of the pulse pressure waveform of FIG. 6.

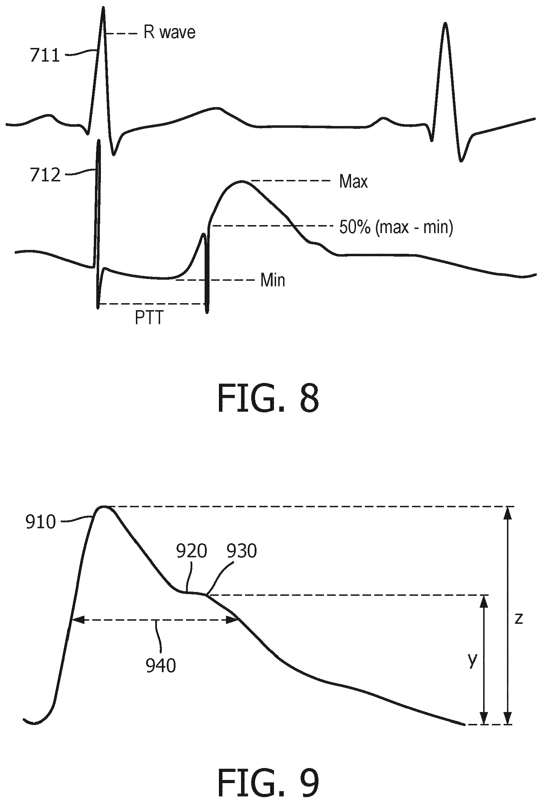

During the inspiration phase, a blood pressure information is also shown in area 755 in FIG. 6. The blood pressure information may be computed in accordance with the aforementioned embodiments. The blood pressure information may be derived, for example, from PPG sensor signals, with features characterized as set forth in FIG. 9. Taking one section into consideration, the PGG sensor signals may be converted into a waveform that includes a systolic peak 910, a dicrotic notch 920, and a diastolic peak 930. The amplitude of the systolic peak may be given by X, the amplitude of the diastolic peak may be given by Y, and the peak may have a width 940. Further, as illustrated in FIG. 6, the diastolic pressure may correspond to negative peak 761, systolic pressure may correspond to positive peak 762, a boundary 763 may corresponding to a partition between diastolic and systolic phases with corresponding shaded portions 764 and 765 averaged (e.g., integrated) over time. In one embodiment, blood pressure may be determined from a single or average of several observations of data obtained that correlates to the blood pressure in one respiratory phase or a series of averages or of median values of pulsations across several (typically adjacent) phases.

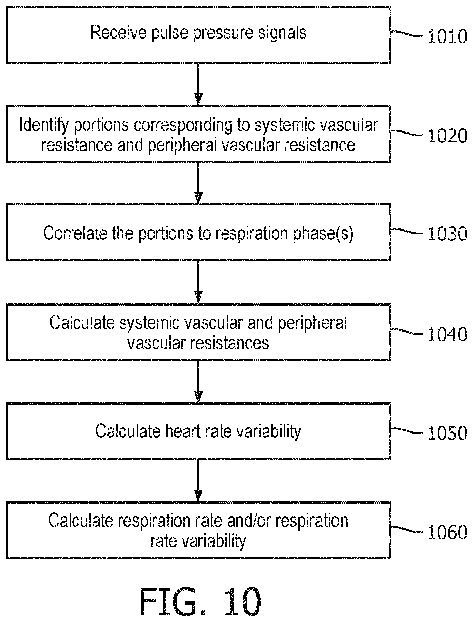

Referring to FIGS. 6 and 10, the method may detect the vital sign of heart rate variability. The method may be performed, in whole or part, for example, by the system of FIG. 2 or may be performed by another method. At 1010, the method includes receiving physiological signals that include, for example, pulse pressure signals of a patient detected over time.

At 1020, the pulse pressure signals may be processed to identify one or more predetermined characteristics from the pulse pressure signals. These characteristics may include portions of a waveform indicative of systemic vascular resistance 1050 and portions of a waveform indicative of peripheral vascular resistance 1060.

At 1030, the portions of the waveforms that are indicative of systemic vascular resistance and peripheral vascular resistance may be correlated to different respiration phases of the patient. In one embodiment, the systemic vascular and peripheral vascular resistances may be detected and correlated to the expiration phase 736 of respiration during an SpO₂ (oxygen saturation) period and/or a COHb (carboxyhemoglobin) period.

At 1040, the systemic vascular resistance may be calculated by measuring the areas under each waveform 1050 defined by a boundary 1055 corresponding to a peak and then taking a ratio of these areas to generate a measurement of systemic vascular resistance. Similarly, the peripheral vascular resistance may be calculated by measuring the areas under each waveform 1060 defined by a boundary 1065 corresponding to a peak and then taking a ratio of these areas to generate a measurement of peripheral vascular resistance.

At 1050, the heart rate variability of the patient may be calculated based on the systemic vascular resistance and peripheral vascular resistance measurements occurring during expiration.

At 1060, the respiration rate and/or the variability in respiration rate of the patient may be calculated based on systemic vascular resistance measurements and peripheral vascular resistance measurements taken over both SpO₂ and COHb periods.

In one embodiment, pulsus paradoxus may be detected based on the blood pressure waveform 755 and the systemic vascular resistance 1050. An example of pulsus paradoxus 1100 is shown in FIG. 6 during the COHb period. Also, in this period, the height of the blood pressure waveform 755 may be indicative of fluid volume 1150, as labeled.

In accordance with one or more of the aforementioned embodiments, a system and method measures vital signals in a way that is more accurate than other methods and which allows a greater amount of information to be extracted that is relevant to patient health. The vital signs may include, but are not limited to, blood pressure, heart rate variability, and pulse transit time as well as other vital signs. In some implementations, these vital signs may be more accurately measured in a way that excludes distortion or other effects caused by pulsus paradoxus. This may be achieved by correlating pulse pressure signals or other physiological signals to at least one of the two phases (expiration and inspiration) of the respiratory cycle, and then measuring the vital signs based on this correlation. The pulse pressure signals may be measured directly (e.g., based on direct pressure of the arterial blood) or indirectly (e.g., based on surrogate pulse measured by auscultation or PPG waveform).

In at least some embodiments, the pulse pressure signals (and/or waveforms generated based on these signals) are correlated to the expiration phase. This may be advantageous for some applications. For example, the time period of expiration is typically twice that which occurs during inspiration. Correlating pulse pressure signals to expiration, therefore, may increase the sample size, which, in turn, may produce a more accurate and reliable measurement. Also, the effect of cardio-pulmonary coupling tends to be minimal during expiration. As a result, the variability of the waveform generated by the pulse pressure signals caused by consequences of disease may be minimal. This, too, may result in the generation of a more accurate measurement.

The blood pressure measurement generated may be based on a single observation of pulse pressure signals, an average of several observations of pulse pressure signals in one respiratory phase, or a series of averages of pulsations across several (e.g., adjacent) phases.

The methods, processes, and operations of the system embodiments described herein may be performed by code or instructions to be executed by a computer, processor, controller, or other signal processing device. The code or instructions may be stored in the non-transitory computer-readable medium as previously described in accordance with one or more embodiments. Because the algorithms that form the basis of the methods (or operations of the computer, processor, controller, or other signal processing device) are described in detail, the code or instructions for implementing the operations of the method embodiments may transform the computer, processor, controller, or other signal processing device into a special-purpose processor for performing the methods herein.

The processors, models, algorithms, detectors, models, and/or other signal, pattern, or data detection, signal generating, or signal processing features of the embodiments disclosed herein may be implemented in logic which, for example, may include hardware, software, or both. When implemented at least partially in hardware, expert systems, processors, detectors, models, or other signal, pattern, or data detection, signal generating, or signal processing features may be, for example, any one of a variety of integrated circuits including but not limited to an application-specific integrated circuit, a field-programmable gate array, a combination of logic gates, a system-on-chip, a microprocessor, or another type of processing or control circuit.

When implemented in at least partially in software, the expert systems, processors, detectors, models, or other signal, pattern, or data detection, signal generating, or signal processing features may include, for example, a memory or other storage device for storing code or instructions to be executed, for example, by a computer, processor, microprocessor, controller, or other signal processing device. The computer, processor, microprocessor, controller, or other signal processing device may be those described herein or one in addition to the elements described herein. Because the algorithms that form the basis of the methods (or operations of the computer, processor, microprocessor, controller, or other signal processing device) are described in detail, the code or instructions for implementing the operations of the method embodiments may transform the computer, processor, controller, or other signal processing device into a special-purpose processor for performing the methods herein.

Although the various exemplary embodiments have been described in detail with particular reference to certain exemplary aspects thereof, it should be understood that the invention is capable of other example embodiments and its details are capable of modifications in various obvious respects. As is readily apparent to those skilled in the art, variations and modifications can be affected while remaining within the scope of the invention. Accordingly, the foregoing disclosure, description, and figures are for illustrative purposes only and do not in any way limit the invention, which is defined only by the independent claims.

## Claims

1. A method for processing information, the method comprising:
receiving (410) physiological signals of a patient;
extracting (430) first information from the physiological signals;
extracting second information (440, 450) from the physiological signals;
correlating (460) the first information to the second information; and
determining (470) a condition of the patient based on the correlation between the first information and the second information, wherein the first information includes a systemic parameter, the second information includes respiration information and the condition is a vital sign of the patient.

2. The method of claim 1, wherein the second information is derived from the first information.

3. The method of claim 2, wherein:
the systemic parameter is pulse pressure;
the respiration information includes at least one of an inspiration phase and an expiration phase of a respiratory cycle; and
the vital sign is a blood pressure measurement.

4. The method of claim 3, wherein the physiological signals include pulse pressure signals.

5. The method of claim 4, wherein correlating the first information to the second information includes determining portions of the pulse pressure signals that correspond to the inspiration phase of the respiratory cycle and wherein determining the condition includes determining the blood pressure measurement based on the portions of the pulse pressure signals that correspond to the inspiration phase.

6. The method of claim 4, wherein correlating the first information to the second information includes determining portions of pulse pressure signals that correspond to the expiration phase of the respiratory cycle and wherein determining the condition includes determining the blood pressure measurement based on the portions of the pulse pressure signals that correspond to the expiration phase.

7. The method of claim 4, wherein correlating the first information to the second information includes calculating differential information based on portions of the pulse pressure signals correlated to the inspiration phase and the expiration phase, and wherein determining the condition includes determining the blood pressure measurement based on the differential information.

8. The method of claim 1, wherein the vital sign is heart rate or heart rate variability.

9. The method of claim 1, wherein:
the vital sign is blood pressure;
the systemic parameter is pulse pressure;
the physiological signals are pulse pressure signals; and
correlating pulse pressure to the respiration information reduces distortion in the determined blood pressure of the patient caused by pulsus paradoxus.

10. The method of claim 9, wherein the pulse pressure signals are received during at least one of an oxygen saturation period or a carboxyhemoglobin period.

11. A system for processing information, comprising:
a memory (220) configured to store instructions; and
a processor (230) configured to execute the instructions in order to carry out the method of any of the preceding claims.

12. A computer program product comprising instructions to carry out the method of any of the preceding claims 1-10.
